# EUROPEAN PATENT SPECIFICATION

(11) **EP 4 550 010 B1**
(45) Date of publication and mention of the grant of the patent: **09.12.2026**
(21) Application number: 24209120.5
(22) Date of filing: 28.10.2024
(51) Int. Cl.: G01V 5/22, G01N 23/083, G01N 33/02

(54) **X-RAY INSPECTION APPARATUS**
RÖNTGENPRÜFGERÄT
APPAREIL D'INSPECTION PAR RAYONS X

(30) Priority: 31.10.2023 JP 2023186660
(43) Date of publication of application: 07.05.2025
(73) Proprietor: Ishida Co., Ltd., Kyoto-shi Kyoto 606-8392 (JP)
(72) Inventor: YURUGI, Futoshi, Ritto-shi, Shiga, 520-3026 (JP); TOMINAGA, Kota, Ritto-shi, Shiga, 520-3026 (JP); KITA, Shojiro, Ritto-shi, Shiga, 520-3026 (JP); ONDA, Satoshi, Ritto-shi, Shiga, 520-3026 (JP)
(74) Representative: Global IP Europe Patentanwaltskanzlei

(56) References cited:
- JP-B2- 6 979 185
- US-A1- 2014 328 463
- US-B2- 8 265 223

## Description

### Technical Field

The present invention relates to an X-ray inspection apparatus.

### Background Art

In the related art, an inspection apparatus using electromagnetic waves such as X-rays is used as an apparatus for inspecting objects to be inspected (articles) such as foodstuffs and pharmaceuticals. For example, an article inspection apparatus disclosed in Patent Document 1 is provided with an irradiation unit that irradiates a moving object to be inspected with radially spreading electromagnetic waves, and a detection unit that detects electromagnetic waves affected by the object to be inspected by detection elements.

US 2014/328463 A1 discloses an X-ray inspection apparatus, comprising:
a conveying unit configured to convey an article;
an irradiation unit configured to irradiate the article conveyed by the conveying unit with X-rays;
a sensor unit having a plurality of detection elements, the plurality of detection elements being configured to detect the X-rays and being arranged in a planar shape; and
an image generation unit configured to read detection results output from at least some of the plurality of detection elements and generate an image.

### Citation List

### Patent Literature

Patent Document 1: JP 2021-156635 A

### Summary of Invention

### Technical Problem

In the article inspection apparatus as described above, there is a tendency that the irradiation unit which is an output source of the electromagnetic waves deteriorates at the earliest. To suppress the deterioration of the irradiation unit, it is conceivable to suppress the output of the electromagnetic waves. However, when the output of the electromagnetic waves is simply suppressed, the detection of the electromagnetic waves by the detection unit becomes insufficient, and there is a concern that the pass/fail judgement of an object to be inspected may not be accurately conducted.

An object of one aspect of the present invention is to provide an X-ray inspection apparatus that can perform a satisfactory inspection regardless of suppressing the output of X-rays.

### Solution to Problem

(1) An X-ray inspection apparatus according to one aspect of the present invention, includes a conveying unit configured to convey an article, an irradiation unit configured to irradiate the article conveyed by the conveying unit with X-rays, a sensor unit in which detection elements configured to detect the X-rays are arranged in a planar shape, and an image generation unit configured to read detection results output from at least some of the detection elements and generate an image. The X-ray inspection apparatus is characterized by a mode switching unit configured to switch an operation mode between a first mode in which a first electric power is supplied to the irradiation unit and a second mode in which a second electric power less than the first electric power is supplied to the irradiation unit. The mode switching unit changes, when switching the operation mode from the first mode to the second mode, a read frequency at which the image generation unit reads the detection results.
   In this X-ray inspection apparatus, when switching the operation mode from the first mode in which the first power is supplied to the irradiation unit to the second mode in which the second power less than the first power is supplied to the irradiation unit, the mode switching unit changes the read frequency at which the image generation unit reads the detection results. For example, it is possible to extend the exposure time of the detection elements during a predetermined period by reducing the read frequency of the detection results, when the first mode is switched to the second mode. This enables satisfactory detection results of the X-rays to be output from each detection element, even when the output of the X-rays emitted from the irradiation unit is weakened in the second mode. Thus, by using the above X-ray inspection apparatus, a satisfactory inspection can be performed regardless of suppressing the output of X-rays.
(2) In the X-ray inspection apparatus mentioned above, the sensor unit may be configured to detect the X-rays by a photon counting method. In this case, the contrast of the image generated by the image generation unit can be improved.
(3) In any one of the X-ray inspection apparatuses mentioned above, the second electric power may be any multiple less than 1 time of the first power. In this case, the power consumption of the X-ray inspection apparatus can be satisfactorily reduced.
(4) In any one of the X-ray inspection apparatuses mentioned above, when switching the operation mode from the first mode to the second mode, the mode switching unit may multiply the read frequency by any multiple less than 1 time. In this case, satisfactory detection results of the X-rays can be surely output from each sensor.
(5) In any one of the X-ray inspection apparatuses mentioned above, when the first mode is switched to the second mode, the sensor unit may change the number of detection elements that operate among the detection elements. In this case, since the detection element with an insufficient exposure time hardly exists, an unclear image is less likely to be generated.
(6) In any one of the X-ray inspection apparatuses mentioned above, when the first mode is switched to the second mode, a change rate of the read frequency may be the same as a ratio of the detection elements that operate among the detection elements. In this case, satisfactory detection results of the X-rays can be surely output from operating detection elements.
(7) Any one of the X-ray inspection apparatuses mentioned above may further include a determination unit configured to determine whether a foreign matter is present in the article including a food, on the basis of the image. In this case, the determination unit can satisfactorily determine whether a foreign matter is present even when the X-ray output is suppressed.

### Advantageous Effects of Invention

One aspect of the present invention can provide an X-ray inspection apparatus that can perform a satisfactory inspection regardless of suppressing the output of X-rays.

### Brief Description of Drawings

FIG. 1 is a configuration diagram of an X-ray inspection apparatus according to an embodiment.
FIG. 2 is a schematic configuration diagram of the interior of a shield box illustrated in FIG. 1.
FIG. 3 is a schematic diagram illustrating detection elements in operation and detection elements not used for image generation.
FIG. 4 is a functional configuration diagram of a control unit.

### Description of Embodiments

A preferred embodiment of the present invention will now be described in detail with reference to the accompanying drawings. Note that in the description of the drawings, the same or equivalent elements are denoted by the same reference signs, and duplicate descriptions will be omitted.

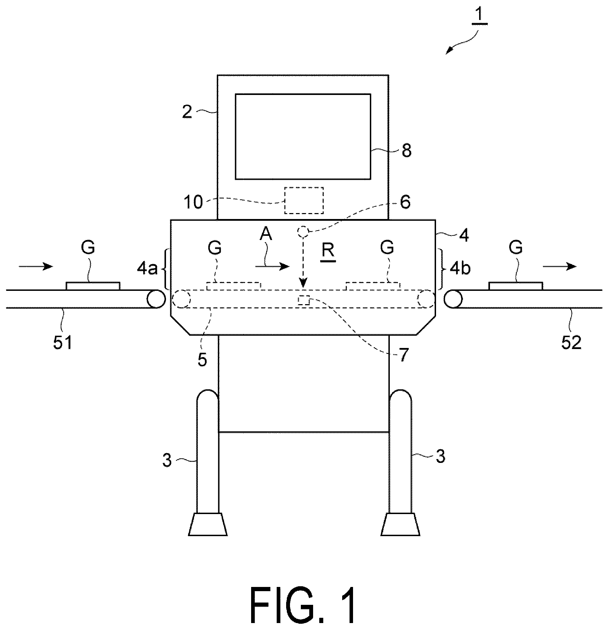

As illustrated in FIG. 1, an X-ray inspection apparatus 1 includes a main body 2, a support leg 3, a shield box 4, a conveying unit 5, an X-ray irradiation unit 6, a sensor unit 7, a display operation unit 8, and a control unit 10. The X-ray inspection apparatus 1 generates X-ray transmission images of an article G while conveying the article G, and inspects the article G on the basis of the X-ray transmission images. The article G before inspection is carried into the X-ray inspection apparatus 1 by a supply conveyor 51. The article G after inspection is carried out from the X-ray inspection apparatus 1 by a delivery conveyor 52.

The main body 2 accommodates the control unit 10 or the like. The support leg 3 supports the main body 2. The shield box 4 is provided in the main body 2. The shield box 4 is a housing for preventing leakage of X-rays (electromagnetic waves) to the outside. Inside the shield box 4, an inspection chamber R is provided in which the inspection of article G by X-rays is performed. A supply port 4a and a delivery port 4b are formed in the shield box 4. The article G before the inspection is carried from the supply conveyor 51 to the inspection chamber R through the supply port 4a. The article G after the inspection is carried from the inspection chamber R to the delivery conveyor 52 through the delivery port 4b.

The conveying unit 5 is a member for conveying the article G, and is arranged extending through the center of the shield box 4. The conveying unit 5 conveys the article G along a conveyance direction A from the supply port 4a to the delivery port 4b via the inspection chamber R. The speed (conveyance speed) at which the article G is conveyed by the conveying unit 5 is set by the control unit 10, for example. The conveying unit 5 is, for example, a belt conveyor stretched between the supply port 4a and the delivery port 4b. Note that the conveying unit 5 may project outward from the supply port 4a and the delivery port 4b.

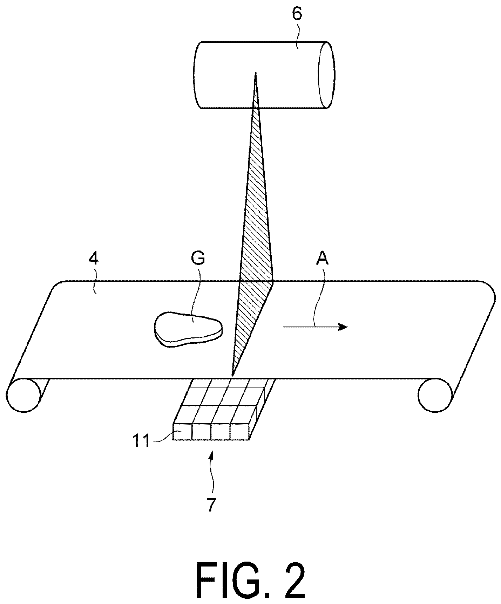

As illustrated in FIGS. 1 and 2, the X-ray irradiation unit 6 is an electromagnetic waves irradiation unit arranged in the shield box 4, and irradiates the article G conveyed by the conveying unit 5 with X-rays. The X-rays include X-rays of various energy regions from low energy (long wavelength) to high energy (short wavelength). Thus, the X-ray irradiation unit 6 irradiates the article G conveyed by the conveying unit 5 with X-rays of a plurality of energy regions. X-ray irradiation (i.e., idling of X-ray irradiation unit 6) by the X-ray irradiation unit 6 may be performed after the activation of the X-ray inspection apparatus 1 and before the inspection of the article G. Note that "low" and "high" in the above-described low energy and high energy indicate "low" and "high" relatively among a plurality of energy regions of the X-rays emitted from the X-ray irradiation unit 6, and do not indicate a specific range.

In the present embodiment, the power (in particular, current) supplied to the X-ray irradiation unit 6 can be changed manually or automatically. By changing the power, the output of the X-rays emitted to the article G can be changed. For example, when the sensor unit 7 is irradiated with excessive X-rays, the power supplied to the X-ray irradiation unit 6 is reduced. On the other hand, when the sensor unit 7 is irradiated with an insufficient amount of X-rays, the power supplied to the X-ray irradiation unit 6 is increased. When an operation mode or the like of the X-ray inspection apparatus 1 is manually or automatically set, electric power (particularly, current) supplied to the X-ray irradiation unit 6 may be switched. For example, when a normal mode (first mode) and a power-saving mode (second mode) are included in the operation mode, a first power is supplied to the X-ray irradiation unit 6 in the normal mode, and a second power less than the first power is supplied to the X-ray irradiation unit 6 in the power-saving mode. The second power is, for example, a reciprocal multiple of a natural number of the first power, but not limited to this. For example, the second power may be any multiple (0.9 times, 0.85 times, 0.6 times, 0.3 times, or the like) less than 1 time of the first power. In the power-saving mode, the article G is inspected in a state where the electric power supplied to the X-ray inspection apparatus 1 is reduced as compared with in the normal mode. In the present specification, a natural number is an integer of 2 or more.

The sensor unit 7 is a sensor unit that detects electromagnetic waves. The sensor unit 7 is arranged in the shield box 4 at a position facing the X-ray irradiation unit 6 in the vertical direction. The sensor unit 7 includes detection elements 11 arranged in a planar shape (two-dimensional). The detection elements 11 are arranged in a direction (width direction) orthogonal to at least the conveyance direction A of the conveying unit 5 and the vertical direction. A line sensor may be configured by some of the detection elements 11 in the sensor unit 7. For example, line sensors extending along the width direction may be arranged side by side in the conveyance direction A in the sensor unit 7. In this case, each of the line sensors includes detection elements 11 arranged along the width direction. Note that unless otherwise mentioned below, the detection elements 11 correspond to all the detection elements 11 included in the sensor unit 7.

In the present embodiment, the sensor unit 7 is a direct conversion type detection unit that can detect X-rays by a photon counting method. The detection element 11 is, for example, a sensor (multi-energy sensor) that detects X-rays in each of a plurality of energy regions transmitted through the article G, and the sensor unit 7 may be a Time Delay Integration sensor (TDI sensor). The detection element 11 includes, for example, a photon detection type sensor such as a CdTe semiconductor detector. In the detection element 11, for example, an electron-hole pair is generated when an X-ray photon arrives. A process for counting photons (photon counting) is performed on the basis of the energy (photon energy) obtained at this time. The counting process is performed, for example, by the sensor unit 7 or by a calculation unit (not illustrated) included in the detection element 11. The result of the counting process (detection result) of each detection element 11 by the calculation unit is output to the control unit 10 at a predetermined time interval, for example. The predetermined time interval is also referred to as a read interval or a delay time, and can be appropriately changed by the control unit 10 (details will be described below).

Each detection element 11 of the sensor unit 7 may classify the photon energy of X-rays, which is detected on the basis of any threshold, into two or more energy regions. In this case, the sensor unit 7 can perform photon counting in each energy region. Any threshold is, for example, one or more values (in keV) set by the control unit 10. Any threshold may be set, for example, by the method described in JP 2023-132587 A, or by using a following brightness level.

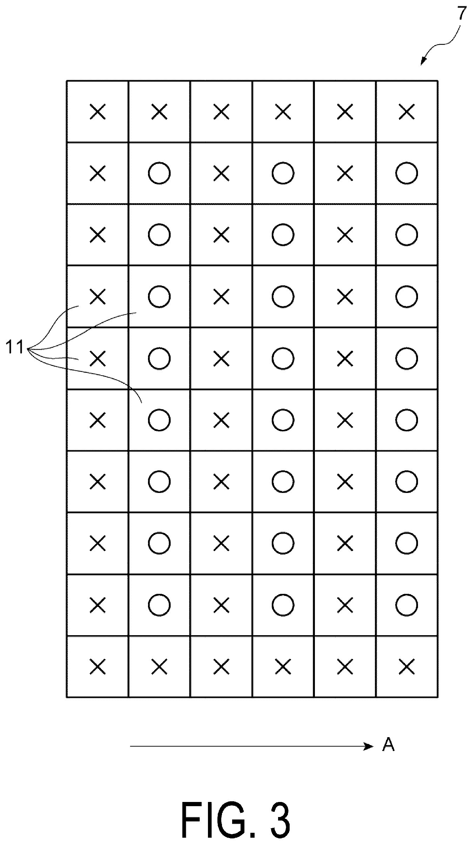

The sensor unit 7 may change the number of operating detection elements 11 on the basis of the conveyance speed of the conveying unit 5, the operation mode of the X-ray inspection apparatus 1, or the like. FIG. 3 is a schematic diagram illustrating operating detection elements, and detection elements not used for image generation. FIG. 3 illustrates some of the detection elements 11 included in the sensor unit 7, and "○" is added to each of operating detection elements 11 (detection elements 11 used for image generation), and "×" is added to each of detection elements 11 not used for image generation. For example, when the conveyance speed is excessive and satisfactory detection results cannot be output from all the detection elements 11, or when the control unit 10 switches the operation mode of the X-ray inspection apparatus 1 from the normal mode to the power-saving mode, the sensor unit 7 limits the number of operating detection elements 11 as illustrated in FIG. 3. Thus, satisfactory detection results can be output from all the operating detection elements 11. The detection elements 11 to be limited are selected according to predetermined rules, patterns, or the like. The rules, patterns, or the like may be appropriately changed depending on the type of article G, the total operation time of the X-ray inspection apparatus 1, or the like. For example, when the X-ray inspection apparatus 1 operates in the second mode, the operating detection elements 11 may be always the same or may be changed depending on the cumulative operation time of the X-ray inspection apparatus 1. On the other hand, when the sensor unit 7 limits the number of operating detection elements 11 and when the conveyance speed is no longer excessive, the sensor unit 7 releases the limit on the number of operating detection elements 11. This increases the number of operating detection elements 11. Note that when the line sensors are arranged in the sensor unit 7, the sensor unit 7 may limit the number of operating line sensors. In this case, control of the detection elements 11 by the sensor unit 7 can be simplified. When the X-ray inspection apparatus 1 operates in the second mode, every other line sensor of the line sensors included in the sensor unit 7 in the conveyance direction A may operate or every two line sensors may operate, for example. Note that in the present embodiment, the sensor unit 7 changes the number of operating detection elements 11, but not limited to this. The number of operating detection elements 11 may be changed manually, for example.

When the X-ray inspection apparatus 1 is activated, sensitivity correction (calibration) of the sensor unit 7 may be performed. In the sensitivity correction, a brightness level of each detection element 11 is acquired on the basis of the X-rays that have not passed through the article G. The brightness level is a count number of photons detected when the X-rays not passing through the article G are incident on the sensor unit 7. Sensitivity correction of the sensor unit 7 corresponds to correction of output differences between the detection elements 11 included in the sensor unit 7. From the viewpoint of shortening the start-up time of the X-ray inspection apparatus 1, in the sensitivity correction, X-ray irradiation by the X-ray irradiation unit 6 may be started simultaneously with the start of X-ray detection by the sensor unit 7. Note that the sensitivity correction of the sensor unit 7 is performed by the method described in JP Application No. 2023-39353, for example.

As illustrated in FIG. 1, the display operation unit 8 is a member (display unit) provided in the main body 2. The display operation unit 8 displays various information and receives input operations of various conditions from the outside. The display operation unit 8 is, for example, a liquid crystal display and displays an operation screen as a touch panel. In this case, the operator can input various conditions via the display operation unit 8. For example, the operator can set the operation mode of the X-ray inspection apparatus 1, the conveyance speed of the conveying unit 5, the power (at least one of current and voltage) supplied to the X-ray irradiation unit 6, the number of operating detection elements 11, or the like, through the display operation unit 8. The input operation received by the display operation unit 8 is output to the conveying unit 5, the sensor unit 7, the control unit 10, or the like.

The control unit 10 is arranged inside the main body 2. The control unit 10 controls the operation of each unit of the X-ray inspection apparatus 1. The control unit 10 is composed of a Central Processing Unit (CPU), a Read Only Memory (ROM), a Random Access Memory (RAM), or the like. The ROM stores a program for controlling the X-ray inspection apparatus 1, an operation mode of the X-ray inspection apparatus 1, or the like.

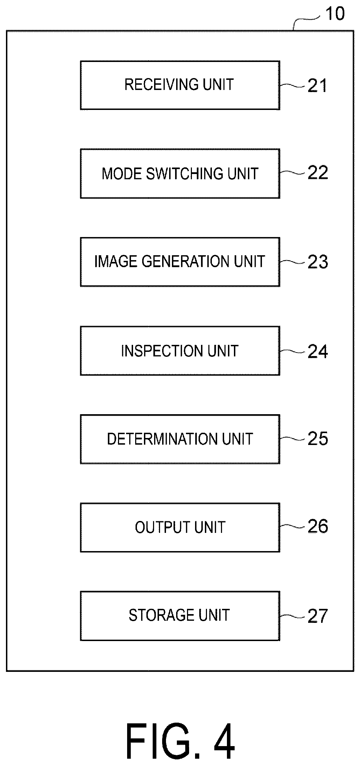

FIG. 4 is a functional configuration diagram of the control unit. As illustrated in FIG. 4, the control unit 10 includes a receiving unit 21, a mode switching unit 22, an image generation unit 23, an inspection unit 24, a determination unit 25, an output unit 26, and a storage unit 27.

The receiving unit 21 receives the input operation received by the display operation unit 8. The receiving unit 21 transmits, for example, a signal (mode designation signal) designating the operation mode of the X-ray inspection apparatus 1 set via the display operation unit 8 to the mode switching unit 22 and the output unit 26. The mode designation signal transmitted to the output unit 26 is output to the sensor unit 7. The receiving unit 21 also receives the detection results output from the sensor unit 7. The receiving unit 21 transmits the received detection results to the image generation unit 23.

The mode switching unit 22 switches the operation mode of the X-ray inspection apparatus 1 to an operation mode according to the mode designation signal. For example, the mode switching unit 22 switches the operation mode between the normal mode in which the first power is supplied to the X-ray irradiation unit 6 and the power-saving mode in which the second power is supplied to the X-ray irradiation unit 6. For example, when switching the operation mode from the normal mode to the power-saving mode, the mode switching unit 22 changes the read frequency (unit: times/hour) at which the image generation unit 23 reads the detection results. The read frequency corresponds to the reciprocal of the above-described read interval. Thus, in the present embodiment, when switching the operation mode from the normal mode to the power-saving mode, the mode switching unit 22 changes the read interval of the detection results by the image generation unit 23.

In the present embodiment, when the mode switching unit 22 switches the operation mode from the normal mode to the power-saving mode, not only the power supplied to the X-ray irradiation unit 6 is reduced, but also the mode switching unit 22 reduces the read frequency at which the image generation unit 23 reads the detection results. This enables the period (X-ray exposure period) during which the detection elements 11 are exposed to X-rays in the read interval to be extended. The mode switching unit 22 may also change the read frequency at which the image generation unit 23 reads the detection results, when switching the operation mode from the power-saving mode to the normal mode. This makes it difficult for the detection elements 11 to detect excessive X-rays. In the present embodiment, the mode switching unit 22 multiplies the read frequency by the reciprocal of a natural number when switching the operation mode from the normal mode to the power-saving mode, but not limited to this. For example, when switching the operation mode from the normal mode to the power-saving mode, the mode switching unit 22 may multiply the read frequency by any multiple (e.g., 0.75 times, 0.7 times, 0.6 times, 0.4 times, or the like) less than 1 time. In other words, when switching the operation mode from the normal mode to the power-saving mode, the mode switching unit 22 may multiply the read interval by a natural number, or may multiply the read interval by any multiple (1.25 times, 1.4 times, 1.6 times, 1.75 times, or the like) more than 1 time. Regardless of the change in the read interval, the period required for the counting process by the sensor unit 7, or by the calculation unit included in the detection element 11 is constant. Thus, regardless of the operation mode of the X-ray inspection apparatus 1, the read interval may be changed such that the ratio of the X-ray exposure period of each detection element 11 in the read interval becomes a predetermined value or more.

When the mode switching unit 22 switches the operation mode from the normal mode to the power-saving mode, both the change of the read frequency and the change of the number of the operating detection elements 11 by the sensor unit 7 may be performed. In this case, the change rate of the read frequency may have some relationship with the ratio of the operating detection elements 11 among all the detection elements 11 included in the sensor unit 7. For example, the change rate of the read interval may be a reciprocal multiple of the ratio of the operating detection elements 11 among the detection elements 11, or may be a ratio other than a reciprocal multiple of the ratio. When the change rate of the read frequency is the same as the ratio of the operating detection elements 11 among the detection elements 11, the operation control of the X-ray inspection apparatus 1 can be facilitated.

The image generation unit 23 is mainly composed of, for example, a Graphics Processing Unit (GPU), and develops the detection result signals into a two-dimensional image on a memory. The memory in which the two-dimensional image is developed is, for example, a memory included in the GPU, but not limited to this. The image generation unit 23 reads the detection results output from at least some of the detection elements 11 included in the sensor unit 7 at the read interval, for example, to generate one or more time delay integral images used for the inspection of the article G. For example, transmission images respectively corresponding to the plurality of energy regions are generated by the image generation unit 23. The image generation unit 23 may generate one or more of difference images from the transmission images. The image generation unit 23 may use, for example, an image processing algorithm or a program automatically set by machine learning. The image processing algorithm is composed of one image processing filter or a combination of several image processing filters. At least one or more of image processing algorithms can be automatically generated from the image processing filters on the basis of the specifications, inspection conditions or the like of the X-ray inspection apparatus 1 by employing Genetic Algorithms (GA), which are methods realized by applying the mechanism of heredity and evolution in the biological world. At least a part of the image processing algorithms can also be appropriately set by the operator via the display operation unit 8. The program automatically set by machine learning is a predictive model (learned model) generated by machine learning, and an inference program in which parameters (learned parameters) obtained as a result of machine learning are incorporated. Examples of machine learning used in the learned model include neural networks, support vector machines, and genetic algorithms.

The inspection unit 24 inspects the article G on the basis of the images generated by the image generation unit 23. For example, the inspection unit 24 inspects the article G by using the transmission images, the difference images, or the like. The inspection unit 24 may inspect the article G on the basis of both the difference images and the transmission images. During the generation of the difference images by the image generation unit 23, the inspection of the article G based on the transmission images or the like may be performed. The inspection unit 24 inspects, for example, the article G for whether a foreign matter is present or whether a chip/crack is present, but not limited to this. In the case where the article G is wrapped in a sheet-like packaging material, the inspection unit 24 can also inspect a break of the packaging material, a seal failure of the packaging material (product in seal), or the like. In the case where the article G is accommodated in a package, the inspection unit 24 can perform a foreign matter confirmation inspection in the package, a missing part confirmation inspection, a number of accommodated articles confirmation inspection, a cavity confirmation inspection, or the like. The inspection unit 24 transmits the inspection results of the article G to the determination unit 25 and the storage unit 27.

The determination unit 25 determines whether the article G is non-defective on the basis of the inspection results received from the inspection unit 24. For example, the determination unit 25 determines whether a foreign matter is present in the article G, whether a chip/crack is present in the article G, or the like. The determination unit 25 transmits the determination results to the output unit 26 and the storage unit 27.

The output unit 26 outputs the determination results of the determination unit 25 to at least one of the units other than the control unit 10 in the X-ray inspection apparatus 1, and the apparatuses different from the X-ray inspection apparatus 1. This enables at least either of the X-ray inspection apparatus 1 and the apparatuses different from the X-ray inspection apparatus 1 (e.g., a sorting apparatus located downstream of the X-ray inspection apparatus 1) to perform the operation when the article G is defective. Other examples of the apparatuses different from the X-ray inspection apparatus 1 include, for example, the supply conveyor 51, the delivery conveyor 52, and a notification apparatuses.

The storage unit 27 stores signals, data, or the like generated by the control unit 10. For example, the storage unit 27 stores detection results transmitted from the receiving unit 21, image data transmitted from the image generation unit 23, data related to inspection results transmitted from the inspection unit 24, and data related to determination results transmitted from the determination unit 25.

In the X-ray inspection apparatus 1 according to the present embodiment described above, when the mode switching unit 22 switches the operation mode from the normal mode in which the first power is supplied to the X-ray irradiation unit 6 to the power-saving mode in which the second power is supplied to the X-ray irradiation unit 6, the mode switching unit 22 changes the read frequency at which the image generation unit 23 reads the detection results. For example, since the read frequency of the detection results is reduced when the mode switching unit 22 switches the operation mode from the normal mode to the power-saving mode, the period (X-ray exposure period) can be extended during which each detection element 11 is exposed to X-rays in the above-described read interval. This enables satisfactory detection results of the X-rays to be output from each detection element 11 in the read interval, even when the output of the X-rays emitted from the X-ray irradiation unit 6 is weakened in the power-saving mode. Thus, by using the X-ray inspection apparatus 1 according to the present embodiment, a satisfactory inspection can be performed regardless of suppressing the output of X-rays.

In the present embodiment, the sensor unit 7 may detect X-rays by a photon counting method. In this case, the contrast of the image generated by the image generation unit 23 can be improved.

In the present embodiment, the second power may be a reciprocal multiple of a natural number of the first power. In this case, the power consumption of the X-ray inspection apparatus 1 can be satisfactorily reduced.

In the present embodiment, when switching the operation mode from the normal mode to the power-saving mode, the mode switching unit 22 may multiply the read frequency by the reciprocal of a natural number. In this case, the satisfactory detection results of the X-rays can be surely output from each detection element 11.

In the present embodiment, when the normal mode is switched to the power-saving mode, the sensor unit 7 may change the number of operating detection elements 11. In this case, since the detection element 11 with an insufficient X-ray exposure time hardly exists, an unclear image is less likely to be generated.

In the present embodiment, when the normal mode is switched to the power-saving mode, the change rate of the read frequency may be the same as the ratio of the operating detection elements 11 among the detection elements 11. In this case, satisfactory detection results of the X-rays can be surely output from each operating detection element 11.

The X-ray inspection apparatus 1 according to the present embodiment, includes the determination unit 25 that determines whether a foreign matter is present in the article G including a food, on the basis of the image. Thus, the determination unit 25 can satisfactorily determine whether a foreign matter is present even when the output of the X-ray is suppressed.

Although the embodiments of the present invention have been described above, the present invention is not necessarily limited to the above-described embodiments, and various modifications can be made without departing from the gist thereof. For example, in the above-described embodiments, the sensor unit is a device that can detect X-rays by a photon counting method, but not limited thereto. The detection element included in the sensor unit may include at least a scintillator and a photodiode.

In the embodiment described above, the operation mode of the X-ray inspection apparatus includes one normal mode and one power-saving mode, but not limited to this. For example, the operation mode may include a plurality of normal modes. In each of the plurality of normal modes, for example, the power supplied to the X-ray irradiation unit is different from each other. The above operation mode may include a plurality of power-saving modes. In each of the plurality of power-saving modes, for example, at least one of the power supplied to the X-ray irradiation unit and the ratio of operating detection elements is different from each other.

### Reference Signs List

1 X-ray inspection apparatus, 3 Support leg, 4 Shield box, 4a Supply port, 4b Delivery port, 5 Conveying unit, 6 X-ray irradiation unit, 7 Sensor unit, 8 Display operation unit, 10 Control unit, 11 Detection element, 21 Receiving unit, 22 Mode switching unit, 23 Image generation unit, 24 Inspection unit, 25 Determination unit, 26 Output unit, 27 Storage unit, A Conveyance direction, G Article

## Claims

1. An X-ray inspection apparatus (1), comprising:
a conveying unit (5) configured to convey an article (G);
an irradiation unit (6) configured to irradiate the article (G) conveyed by the conveying unit (5) with X-rays;
a sensor unit (7) having a plurality of detection elements (11), the plurality of detection elements (11) being configured to detect the X-rays and being arranged in a planar shape; and
an image generation unit (23) configured to read detection results output from at least some of the plurality of detection elements (11) and generate an image;
**characterized by**
a mode switching unit (22) configured to switch an operation mode between a first mode in which a first electric power is supplied to the irradiation unit (6) and a second mode in which a second electric power less than the first electric power is supplied to the irradiation unit (6), wherein
the mode switching unit (22) is configured to change, when switching the operation mode from the first mode to the second mode, a read frequency at which the image generation unit (23) reads the detection results.

2. The X-ray inspection apparatus (1) according to claim 1, wherein
the sensor unit (7) is configured to detect the X-rays by a photon counting method.

3. The X-ray inspection apparatus (1) according to claim 1 or 2, wherein
the second electric power is any multiple less than 1 time of the first power.

4. The X-ray inspection apparatus (1) according to claim 1, 2 or 3, wherein
when switching the operation mode from the first mode to the second mode, the mode switching unit (22) multiplies the read frequency by any multiple less than 1 time.

5. The X-ray inspection apparatus (1) according to any one of claims 1 to 4, wherein
when the first mode is switched to the second mode, the sensor unit (7) changes the number of detection elements (11) that are to be in operation among the plurality of detection elements (11).

6. The X-ray inspection apparatus (1) according to any one of claims 1 to 5, wherein
when the first mode is switched to the second mode, a change rate of the read frequency corresponds to a ratio of the detection elements (11) that are to be in operation to the plurality of detection elements (11).

7. The X-ray inspection apparatus (1) according to any one of claims 1 to 6, further comprising:
a determination unit (25) configured to determine whether a foreign matter is present in the article (G) including a food, on the basis of the image.

## Patentansprüche

1. Röntgenprüfgerät (1), das aufweist:
eine Fördereinheit (5), die konfiguriert ist, einen Artikel (G) zu fördern;
eine Bestrahlungseinheit (6), die konfiguriert ist, den von der Fördereinheit (5) geförderten Artikel (G) mit Röntgenstrahlen zu bestrahlen;
eine Sensoreinheit (7) mit mehreren Detektionselementen (11), wobei die mehreren Detektionselemente (11) konfiguriert sind, die Röntgenstrahlen zu detektieren, und in einer ebenen Anordnung angeordnet sind; und
eine Bilderzeugungseinheit (23), die konfiguriert ist, von mindestens einigen der mehreren Detektionselemente (11) ausgegebenen Detektionsergebnisse zu lesen und ein Bild zu erzeugen;
**gekennzeichnet durch**
einer Modusumschalteinheit (22), die konfiguriert ist, einen Betriebsmodus zwischen einem ersten Modus, in dem der Bestrahlungseinheit (6) eine erste elektrische Leistung zugeführt wird, und einem zweiten Modus umzuschalten, in dem der Bestrahlungseinheit (6) eine zweite elektrische Leistung zugeführt wird, die geringer ist als die erste elektrische Leistung, wobei
die Modusumschalteinheit (22) konfiguriert ist, beim Umschalten des Betriebsmodus vom ersten Modus in den zweiten Modus eine Lesefrequenz zu ändern, mit der die Bilderzeugungseinheit (23) die Detektionsergebnisse liest.

2. Röntgenprüfgerät (1) nach Anspruch 1, wobei
die Sensoreinheit (7) konfiguriert ist, die Röntgenstrahlen durch ein Photonenzählverfahren zu detektieren.

3. Röntgenprüfgerät (1) nach Anspruch 1 oder 2, wobei
die zweite elektrische Leistung ein beliebiges Vielfaches ist, das kleiner als das Einfache der ersten Leistung ist.

4. Röntgenprüfgerät (1) nach Anspruch 1, 2 oder 3, wobei
beim Umschalten des Betriebsmodus vom ersten Modus in den zweiten Modus die Modusumschalteinheit (22) die Lesefrequenz mit einem beliebigen Vielfachen multipliziert, das kleiner als das Einfache ist.

5. Röntgenprüfgerät (1) nach einem der Ansprüche 1 bis 4, wobei
wenn der erste Modus in den zweiten Modus umgeschaltet wird, die Sensoreinheit (7) die Anzahl der Detektionselemente (11) ändert, die unter den mehreren Detektionselementen (11) in Betrieb sein sollen.

6. Röntgenprüfgerät (1) nach einem der Ansprüche 1 bis 5, wobei
wenn der erste Modus in den zweiten Modus umgeschaltet wird, eine Änderungsrate der Lesefrequenz einem Verhältnis der Detektorelemente (11), die in Betrieb sein sollen, zu den mehreren Detektorelementen (11) entspricht.

7. Röntgenprüfgerät (1) nach einem der Ansprüche 1 bis 6, das ferner aufweist:
eine Bestimmungseinheit (25), die konfiguriert ist, auf der Grundlage des Bildes festzustellen, ob in dem Artikel (G), der ein Lebensmittel enthält, ein Fremdkörper vorhanden ist.

## Revendications

1. Dispositif d'inspection par rayons X (1), comprenant :
une unité de transport (5) prévue pour transporter un article (G) ;
une unité d'irradiation (6) prévue pour irradier l'objet (G) transporté par l'unité de transport (5) avec des rayons X ;
une unité de détection (7) comportant une pluralité d'éléments de détection (11), ladite pluralité d'éléments de détection (11) étant prévue pour détecter les rayons X et étant disposée suivant une forme plane ; et
une unité de génération d'image (23) prévue pour extraire des résultats de détection émis par au moins quelques éléments de la pluralité d'éléments de détection (11) et pour générer une image ;
**caractérisé par**
une unité de commutation de mode (22) prévue pour commuter un mode de fonctionnement entre un premier mode où une première puissance électrique est fournie à l'unité d'irradiation (6) et un deuxième mode où une deuxième puissance électrique inférieure à la première puissance électrique est fournie à l'unité d'irradiation (6), où
l'unité de commutation de mode (22) est prévue pour modifier, lors de la commutation du mode de fonctionnement du premier mode vers le deuxième mode, une fréquence de lecture à laquelle l'unité de génération d'image (23) extrait les résultats de détection.

2. Dispositif d'inspection par rayons X (1) selon la revendication 1, où
l'unité de détection (7) est prévue pour détecter les rayons X par un procédé de comptage de photons.

3. Dispositif d'inspection par rayons X (1) selon la revendication 1 ou la revendication 2, où
la deuxième puissance électrique est un multiple quelconque inférieur à 1 fois la première puissance.

4. Dispositif d'inspection par rayons X (1) selon la revendication 1, la revendication 2 ou la revendication 3, où,
lors de la commutation du premier mode vers le deuxième mode, l'unité de commutation de mode (22) multiplie la fréquence de lecture par un multiple quelconque inférieur à 1.

5. Dispositif d'inspection par rayons X (1) selon l'une des revendications 1 à 4, où, lorsque le premier mode est commuté vers le deuxième mode, l'unité de détection (7) modifie le nombre d'éléments de détection (11) devant être en fonctionnement parmi la pluralité d'éléments de détection (11).

6. Dispositif d'inspection par rayons X (1) selon l'une des revendications 1 à 5, où, lorsque le premier mode est commuté vers le deuxième mode, le taux de variation de la fréquence de lecture correspond au rapport entre les éléments de détection (11) devant être en fonctionnement et la pluralité d'éléments de détection (11).

7. Dispositif d'inspection par rayons X (1) selon l'une des revendications 1 à 6, comprenant en outre :
une unité de détermination (25) prévue pour déterminer, sur la base de l'image, si un corps étranger est présent dans l'article (G) comprenant un produit alimentaire.
